# EUROPEAN PATENT SPECIFICATION

(11) **EP 2 049 062 B1**
(45) Date of publication and mention of the grant of the patent: **26.06.2013**
(21) Application number: 07762316.3
(22) Date of filing: 24.05.2007
(51) Int. Cl.: A61J 1/20

(54) **RECONSTITUTION DEVICE**
REKONSTITUTIONSVORRICHTUNG
DISPOSITIF DE RECONSTITUTION

(30) Priority: 25.05.2006 US 803187 P
(43) Date of publication of application: 22.04.2009
(60) Divisional application: 12180625.1
(73) Proprietor: Bayer HealthCare, LLC, Tarrytown, NY 10591 (US)
(72) Inventor: TUCKWELL, Jonathan David, Cambridge, Cambrigeshire CB4 1YB (GB); DYER, Robert, Somerville, MA 02143 (US); KIVLIN, Robert Owen, Norfolk PE31 6BH (GB); PALMER-FELGATE, John Paul, Hampshire SO22 4JY (GB); AVERY, Matthew Burgess, Berkeley, CA 94704 (US); SKINNER, Kevin, George, Peekskill, NY 10566 (US); KADAMUS, Chris, Jamaica Plain, MA 02130 (US); WOOD, Lee, South Wales CF31 4QP (GB); SCHWAN, Peter, 51373 Leverkusen (DE); ARLETT, Ben, Cambridge, MA 02139 (US)
(74) Representative: Linhart, Angela
(86) International application number: PCT/US2007/069639
(87) International publication number: WO 2007/140238

(56) References cited:
- FR-A- 2 753 624

## Description

### Field of the Invention

The present application relates generally to reconstitution devices. More particularly, the application relates to an improved reconstitution device for connecting a closed receptacle and a container, such as a syringe.

### Description of Related Art

In the domain of drug-packaging, it is known to store a component of a medicinal preparation, such as for example its active ingredient, in a recipient closed by a stopper of relatively non-rigid material, for example of elastomer. A liquid may be introduced into this recipient after perforation of the stopper in order to dissolve the component contained in the recipient or place it in suspension, with a view to obtaining a medicinal preparation in liquid form ready to be administered to the patient.

Traditional devices include a base adapted to cover the neck of the recipient and extending in a flange forming an inner bore while a plunger is adapted to slide in the bore, between a position disengaged with respect to the stopper and an engaged position in which a hollow needle borne by the plunger traverses this stopper. The displacement of the plunger from its disengaged position towards its engaged position is effected manually by an operator (See e.g. FR-A-2753624).

However, traditional devices do not include an actuating mechanism to prevent unwanted use of the device and to facilitate user interaction. Since the reconstitution device is not meant to be reused, unwanted actuation of the device could be wasteful and incur unnecessary costs. Furthermore, traditional devices do not prevent the device from being reused.

Accordingly, it is desirable to develop a reconstitution device that facilitates user interaction by increasing the likelihood that the user follows the proper steps in the reconstitution process, as well as sufficiently preventing the device from being inadvertently actuated or reused.

### SUMMARY

The present invention as claimed is directed to a reconstitution device. In accordance with an exemplary embodiment of the invention, the reconstitution device includes: (i) a receptacle, (ii) a cap including a first end secured to the receptacle, a second end, and an inner bore having a central aperture, (iii) a stopper located between the receptacle and the cap, the stopper including a portion capable of being perforated, (iv) a plunger secured to the second end of the cap, (v) a locking mechanism located in the cap, and (vi) an actuating mechanism.

In accordance with another exemplary embodiment of the invention, the reconstitution device comprises: (i) a receptacle having an opening surrounded by a neck, (ii) a cap including a first end, a second end, and an inner bore having an aperture, the first end being secured to the receptacle, (iii) a stopper located within the opening of the neck, the stopper including a portion capable of being perforated, (iv) a plunger secured to the second end of the cap and being adapted to slide along the inner bore of the cap, the plunger having a shaft, (v) a locking mechanism located between the cap and the stopper for engagement with the shaft of the plunger, and (vi) an actuating mechanism located on the plunger and the cap, wherein when the actuating mechanism is activated, the plunger travels downwardly through the locking mechanism to perforate the stopper.

In accordance with yet another exemplary embodiment of the invention, the reconstitution device comprises: (i) a receptacle, (ii) a cap including a first end, a second end, and an inner bore having a central aperture, the first end being secured to the receptacle, (iii) a stopper located between the receptacle and the cap, the stopper including a portion capable of being perforated, (iv) a plunger secured to the second end of the cap, the plunger including a shaft for perforating the stopper, (v) a locking mechanism located on the plunger comprising a thin material including a tongue capable of flexing in a vertical direction and an aperture that is coaxially aligned with the shaft of the plunger, and (vi) a feedback mechanism for indicating to a user when the plunger has perforated the stopper.

The present invention helps to solve the shortcomings of the prior art by facilitating user interaction by increasing the likelihood that the user follows the proper steps in the reconstitution process. The device further provides an improved means of tamper-proofing than currently available reconstitution devices.

These as well as other aspects and advantages will become apparent to those of ordinary skill in the art by reading the following detailed description, with reference where appropriate to the accompanying drawings. Further, it should be understood that the embodiments described in this summary and elsewhere are intended to illustrate the invention by way of example only.

### BRIEF DESCRIPTION OF THE DRAWINGS

Exemplary embodiments of the invention are described herein with reference to the drawings, in which:
Figure 1 is an expanded view of a reconstitution device of the present invention;
Figure 2 is an expanded view of the plunger shown in Figure 1;
Figure 3 is a cross-sectional view of the reconstitution device of Figure 1;
Figure 4 is a perspective view of the locking mechanism of the device of Figure 1;
Figures 5A - 5C are perspective views of the actuation of the reconstitution device of Figure 1;
Figure 6 is a perspective view of a portion of the reconstitution device of Figure 1;
Figure 7 is a perspective view of the reconstitution device of Figure 1 including a top;
Figures 8A and 8B are perspective views of a second receptacle in use with the reconstitution device of Figure 1;
Figure 9 is a cross-sectional view of a second embodiment of a reconstitution device of the present invention;
Figure 10 is a cross-sectional view of the reconstitution device of Figure 9 including a ratcheting mechanism; and
Figure 11 is a perspective view of the reconstitution device of Figure 9 including a top.

### DETAILED DESCRIPTION

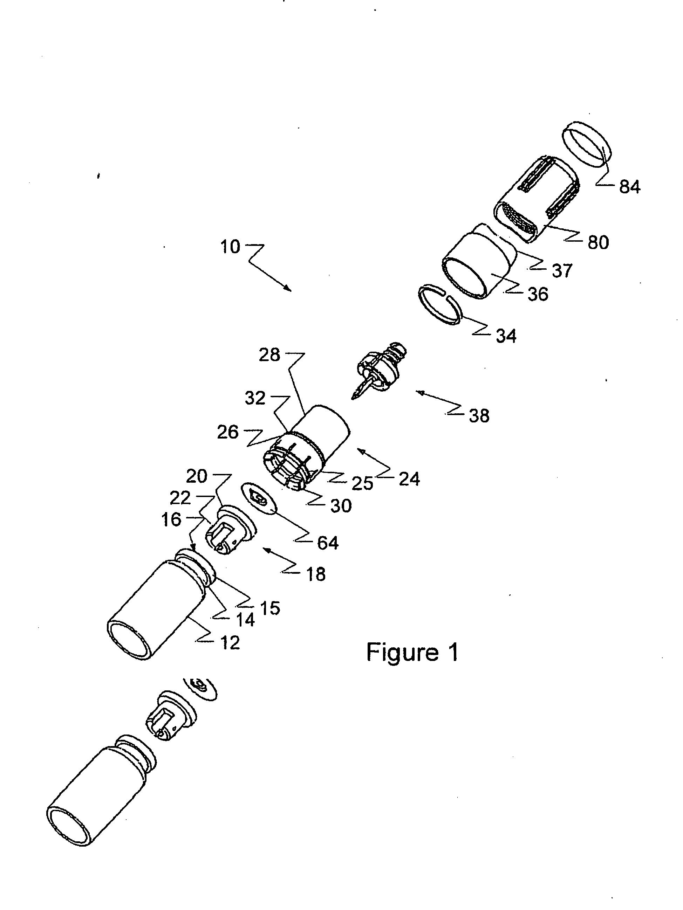

Figure 1 depicts a reconstitution device 10. The device 10 may include a receptacle 12 for storing a first component of a pharmaceutical preparation (not shown), such as its active ingredient, for example. The receptacle 12 may include an opening 16 surrounded or partially surrounded by a neck 14. The neck 14 may also include a lip 15. The opening 16 in the neck 14 allows for a second component (not shown), such as a liquid, to be introduced into the receptacle 12 and mix with the first component. A stopper 18 may be positioned in the opening 16 of the neck 14 to block access to the receptacle 12. The stopper 18 may be made of a relatively non-rigid material, such as elastomer. The stopper 18 may include a top portion 20 located against the lip 15, and a bottom portion 22 located within the opening of the neck 14. The top portion 20 may be capable of being perforated, thereby allowing access to the receptacle 12.

The device 10 may further include a cap 24 secured to the neck 14 of the receptacle 12. The cap 24 may have a first end 26, which is secured to the neck 14 of the receptacle 12, and a second end 28 located opposite the first end 26. The first end 26 of the cap 24 may surround at least a portion of the stopper 18. The first end 26 of the cap 24 may include one or more flexible legs 30 so the cap 24 can expand to fit over the lip 15 of the neck 14 during the manufacturing process, and then contract to securely mate with the neck 14. The cap 24 may further include a step portion 32 located between the first end 26 and the second end 28, thereby separating the first end 26 from the second end 28. The first end 26 of the cap 24 may have a larger diameter than the second end 28 of the cap 24.

The first end 26 of the cap 24 may be secured to the receptacle 12 by a C-clip 34, which may be positioned in an indentation 25 in the first end 26 of the cap 24. Therefore, the C-clip 34 is not located directly on the neck 14 of the receptacle 12 and no scratching occurs. The C-clip 34 may be made of a material not subject to heat degradation, such as metal for example, so that the device 10 may be resistant to heat tampering.

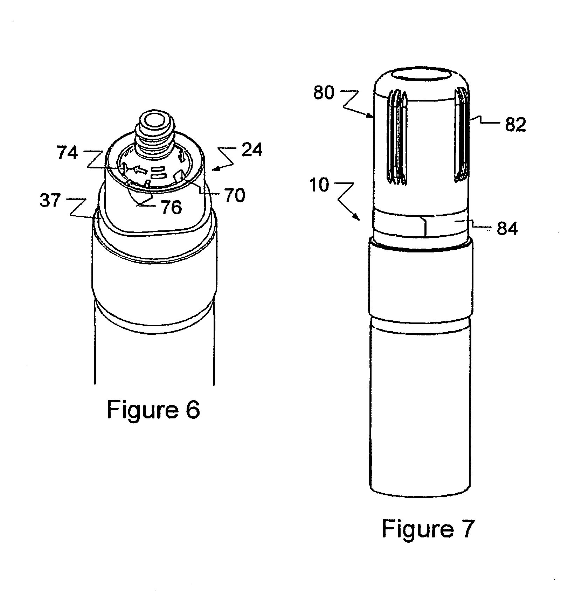

The first end 26 of the cap may be surrounded by a sleeve 36 for protection. The sleeve 36 is prevented from sliding off the cap 24 by the step portion 32. The sleeve 36 may include a wave-shaped part 37 to facilitate opening by a user (not shown), as can be seen in detail in Figure 6. The sleeve 36 may further include a textured surface to facilitate gripping of the device 10 by a user.

Assembly of the first end 26 of the cap 24 to the receptacle 12 may be carried out using various methods. For example, an assembly method may include initially inserting the C-clip 34 into the indentation 25, and then sliding the cap 24 over the stopper 18 and the lip 15. As another example, an assembly method may include initially placing the C-clip 34 onto the second end 28 of the cap 24, then sliding the sleeve 36 over the second end 28 in such a way that the sleeve 36 contacts the C-clip 34 and pushes the C-clip 34 into the indentation 25, and then sliding the cap 24 over the stopper 18 and the lip 15. In accordance with these exemplary methods, as the cap 24 is slid over the stopper 18 and the lip 15, the opening in the C-clip 34 allows the flexible legs 30 to expand as the flexible legs 30 pass over the lip 15, and the flexible legs 30 may contract after passing over the lip 15 to secure the cap to the receptacle 12.

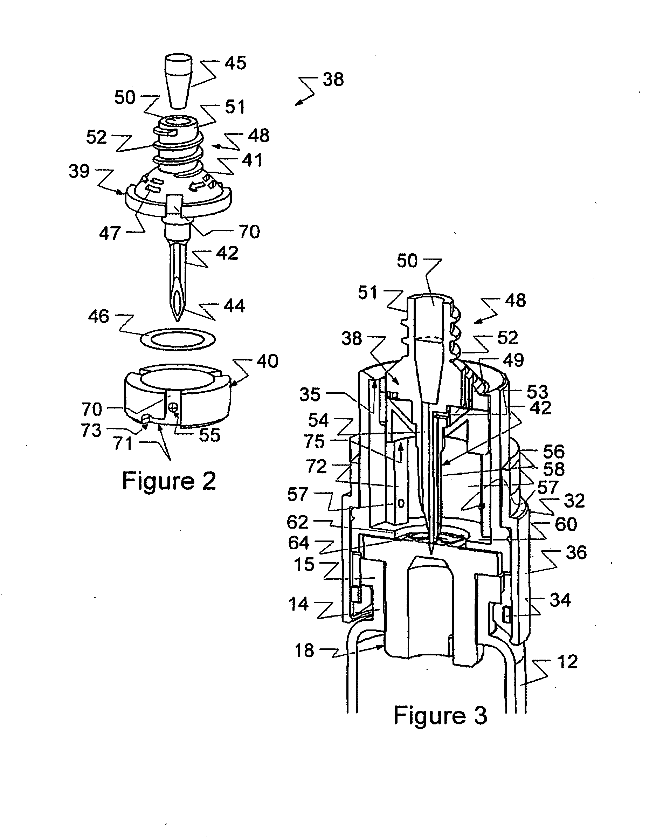

Figure 2 depicts details of a plunger 38 located at the second end 28 of the cap 24. The cap 24 may also include a ridge 35, shown in Figure 3, which may be used to secure the plunger 38 within the cap 24. The plunger 38 may comprise a first portion 39. The first portion 39 of the plunger 38 may include a top surface 41. The top surface 41 may be arranged in various configurations. For example, the top surface 41 may be dome-shaped (i.e., a domed top surface). As another example, the top surface 41 may be angled (i.e., an angled top surface). Other exemplary configurations of the top surface 41 are also possible.

The first portion 39 of the plunger 38 may further include a shaft 42 extending downwardly in a direction towards the stopper 18. The shaft 42 may include a pointed end 44 for piercing the top portion 20 of the stopper 18, thereby allowing the shaft 42 access to the receptacle 12. The pointed end 44 may be angled to cooperate with a locking mechanism 64, which is described in detail below.

In one embodiment, the shaft 42 is elliptical-shaped (e.g., oval shaped). The shaft 42 being elliptical-shaped requires less applied force when the shaft 42 pierces the stopper 18. In alternative embodiments, the shaft 42 may have a cylindrical shape or a rectangular shape.

The plunger 38 may comprise a second portion 40 which is located below the first portion 39. The second portion 40 of the plunger 38 preferably has the same shape as the first portion 39, (e.g., an elliptical shape, a cylindrical shape, or a rectangular shape).

In one exemplary embodiment, the first portion 39 of the plunger 38 is permanently attached to the second portion 40 of the plunger 38 so as to prevent leaks (e.g., a fluid leak) between where the first portion 39 mates to the second portion 40. Various methods may be used to permanently attach the first portion 39 of the plunger 38 to the second portion 40 of the plunger 38. For example, the first portion 39 of the plunger 38 may be permanently attached to the second portion 40 of the plunger 38 by ultrasonic welding. As another example, the first portion 39 of the plunger 38 may be permanently attached to the second portion 40 of the plunger 38 by use of an adhesive (e.g., a glue). Other exemplary methods for permanently attaching the first portion 39 of the plunger 38 to the second portion 40 of the plunger 38 are also possible.

The plunger 38 may further include one or more filtering mechanisms. For example, a fluid filter 45 may be located in the first portion 39 of the plunger so as to filter any liquid that is introduced into the plunger 38. Furthermore, an air filter 46 may be located in a cavity 49 (shown in Figure 3) between the first portion 39 of the plunger 38 and the second portion 40 of the plunger 38 so as to filter any air that is introduced into the receptacle 12. The air filter 46 may be made from any of a variety of materials, but is preferably made of polyethersulphone (PES). Both filters may be located in line with the shaft 42 of the plunger 38.

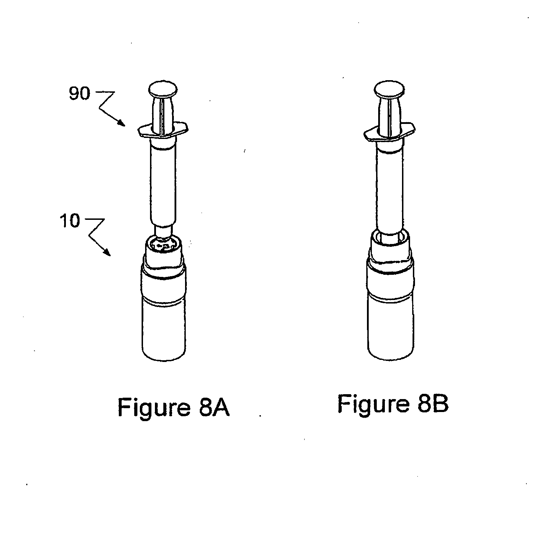

The first portion 39 of the plunger 38 may include a male element 48 extending from the top surface 41 in an upward direction opposite the shaft 42. The male element 48 may be configured to receive a second receptacle 90, such as a syringe, for example, as shown in Figures 8A and 8B. The male element 48 may include an inner bore 50 and an outer surface 51. The outer surface 51 may include a thread 52 for mating with the second receptacle 90.

An advantage of the dome or angled shape of the top surface 41 is that the male element 48 visibly extends above the top surface 41. When the male element 48 receives the second receptacle 90 (e.g., by fastening a threaded syringe to the thread 52 of the male element 48), a user can clearly see when the second receptacle 90 is not in contact with the top surface 41. In this way, a user fastening the second receptacle 90 can continue to turn the second receptacle 90 and clearly tell when the second receptacle 90 makes contact with the top surface 41. Thus, the user knows when second receptacle 90 is fully secured to the male element 48.

Referring to Figure 3, the shaft 42 of the plunger 38 may include a first longitudinal channel 54. The first longitudinal channel 54 establishes communication between the receptacle 12 and the inner bore 50 of the male element 48. The first channel 54 may allow fluid to pass through the shaft 42 of the plunger 38 and into the receptacle 12.

The shaft 42 of the plunger 38 may include a second longitudinal channel 56. The end of the second channel 56 located opposite the pointed end 44 of the shaft 42 may interface to an air channel return 53. The air channel return 53 interfaces to a cavity 49. The cavity 49 is formed by joining the first portion 39 of the plunger 38 to the second portion 40 of the plunger 38. The second channel 56 functions as an air path to allow air to travel out of the receptacle 12 through the shaft 42 of the plunger 38, into the air channel return 53, through the filter 46, and then through the vent 47 located on the top surface 41 of the plunger 38. The air exiting the vent 47 is vented to the atmosphere surrounding the device 10.

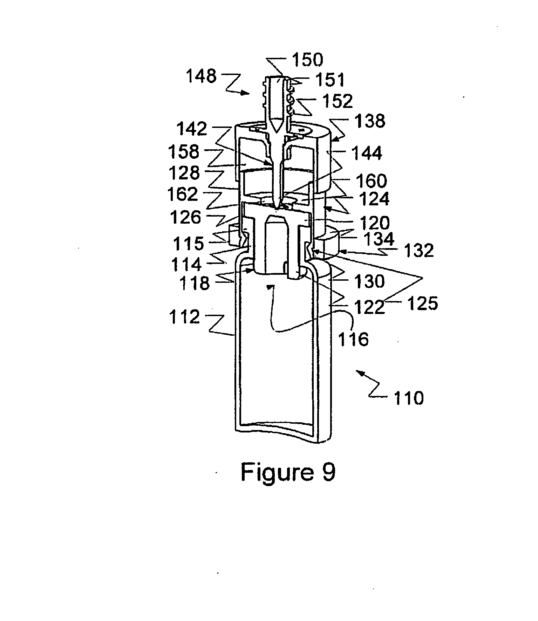

The cap 24 may further include an inner bore 58 having an annular plate 60 with an aperture 62. The aperture 62 is coaxially aligned with the shaft 42 of the plunger 38. The plunger 38 may be configured to slide along the inner bore 58 of the cap 24, or alternatively, the plunger 38 may be configured to slide along an outer surface of the cap, as shown in Figure 9, which will be described below. When the plunger 38 is pushed downward toward the receptacle 12, the shaft 42 moves downwardly through the aperture 62 to pierce the top portion 20 of the stopper 18.

The device 10 may include a locking mechanism 64 for preventing upward movement of the plunger 38 after downward movement of the plunger 38 has occurred. In this manner, the device 10 may be prevented from multiple uses by restraining the plunger 38 in an engaged position, that is, when the shaft 42 pierces the stopper 18.

The locking mechanism 64 may use St. Venant's principle, which states deformations due to stress concentrations are not observed at a distance of three major diameters from the stress concentration. In accordance with this principle, if the locking mechanism 64 is made of a thin material that deforms an area of the shaft 42 greater than the thickness of the thin material as the shaft 42 is moved in a downward direction, then the locking mechanism 64 will prevent the shaft 42 from moving in an upward direction because the locking mechanism will fall in the deformed area of the shaft 42.

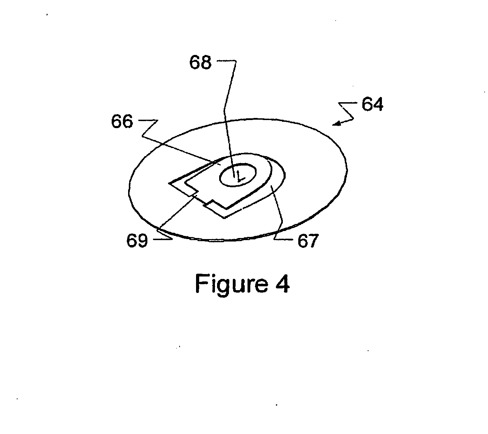

As shown in Figure 4, the locking mechanism 64 may include a thin, cylindrically shaped material. Alternatively, the locking mechanism 64 could be another shape such as elliptical or rectangular. The locking mechanism 64 may be made of any flexible material, such as metal or plastic, for example.

The locking mechanism 64 may be located at various locations within the device 10. For example, as shown in Figure 3, the locking mechanism 64 may be located below the annular plate 60. As another example, the locking mechanism 64 may be located above the annular plate 60. As yet another example, the locking mechanism 64 could be incorporated into the cap 24 (e.g., the locking mechanism could be made of the same material as the cap 24 and molded to the cap 24).

The locking mechanism 64 may include a tongue 66 extending therefrom, and being located within a first aperture 67 of the locking mechanism 64. The tongue 66 may be connected to the first aperture 67 at tab 69, and therefore the tongue 66 is capable of flexing in the vertical direction. The tongue 66 may include a second aperture 68 that is coaxially aligned with the shaft 42. The diameter of the second aperture 68 may be slightly larger than the diameter of the shaft 42. The tongue 66 may be bent upward, as shown in Figure 4.

In operation, the locking mechanism 64 functions as a unidirectional cam. When the plunger 38 is pushed downwardly, the shaft 42 presses the tongue 66 downwardly, thereby opening the second aperture 68 enough to allow the shaft 42 to pass through the second aperture 68 and pierce the stopper 18. The locking mechanism 64 is triggered when a user attempts to pull the plunger 38 upwardly after the plunger 38 has been pushed downwardly.

When the plunger 38 is pulled upwardly, the shaft 42 pulls upwardly on the tongue 66 which causes the tongue 66 to flex vertically in an upward direction. The flexing causes the second aperture 68 to change shape from a first shape (e.g., a cylindrical shape when the second aperture 68 is substantially perpendicular to the shaft 42) to a second shape (e.g., an elliptical shape when the second aperture 68 is in a position not perpendicular to the shaft 42). In this way, the shape of the second aperture 68 may be different from the shape of the shaft 42. Since the shaft 42 cannot freely pass through the second aperture 68 due in part to their different shapes, the tongue 66 then cuts into the shaft 42, creating a stress concentration, or notch (not shown), in the shaft 42. The second aperture 68 in the tongue 66 fits into the notch and prevents the plunger 38 from upward movement. Therefore, the plunger 38 may not be removed from the stopper 18, or moved in an upward direction, without damaging the device 10.

The locking mechanism 64 ensures a smooth downward motion of the plunger 38 with low actuation force and prevents reuse of the device 10 by retaining the plunger 38 in a downward position due to a high retaining force (relative to the actuation force). The locking mechanism 64 may prevent return motion at any point during the downward movement of the shaft 42. Thus, the locking mechanism 64 improves upon retaining clips used in other reconstitution devices to secure a plunger only after the plunger has reached the most downward point of the plunger's downward travel.

The device 10 may include an actuating mechanism. The actuating mechanism may include at least one indentation 70 located on an outer circumference of the plunger 38, on both the first portion 39 and the second portion 40, and at least one protrusion 72 located on the inner bore 58 of the cap 24, on the second end 28 of the cap 24. The indentation 70 located on the second portion 40 may include a passage 71 and a groove 73.

When the device 10 is in a disengaged position, the at least one indentation 70 and the at least one protrusion 72 are not aligned and the plunger 38 is prevented from downward movement. Thus, the actuating mechanism acts as a safety from accidentally pushing down on the plunger 38 and piercing the stopper 18. An upper end 75 of the at least one protrusion 72 may be rounded so that the upper end 75 of the at least one protrusion 72 rests in the groove 73 when the device 10 is in the disengaged position.

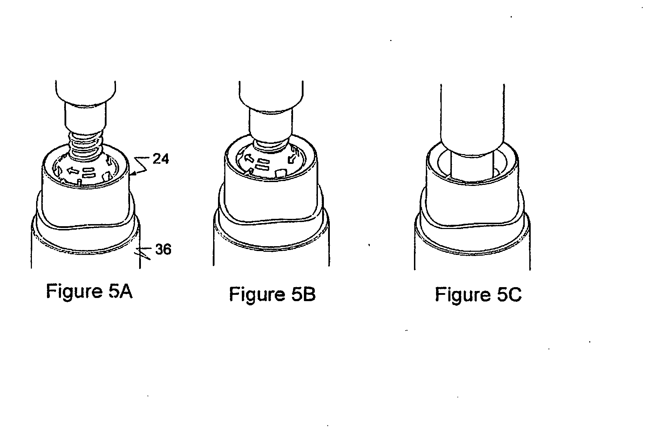

In order to use the device 10, a user may rotate the plunger 38 a given number of degrees until the at least one indentation 70 aligns with the at least one protrusion 72, as shown in Figures 5A and 5B. The top surface 41 of the plunger 38 may include direction markers 74 to indicate to a user which direction to turn the plunger, as best seen in Figure 6. The top surface 41 may further include alignment markers 76 to indicate to the user when the actuating mechanism is aligned.

As the user rotates the plunger 38, the at least one protrusion 72 also moves downward so that the at least one protrusion 72 can move from the groove 73 to the passage 71 and then move from the passage 71 to the at least one indentation 70. The movement from the passage 71 to the at least one indentation 70 can provide tactile feedback and/or audible feedback to the user so that the user knows when the plunger 38 has been rotated the given number of degrees and it is acceptable to push the plunger 38 in a downward direction.

The given number of degrees the plunger 38 is rotated to align the at least one indentation 70 with the at least one protrusion 72 depends on various factors. For example, the factors may include: (i) the number of indentations of the at least one indentation 70 and the number of protrusions of the at least one protrusion 72, (ii) the spacing between each protrusion (if more than one protrusion is used), and the spacing between each indentation (if more than one indentation is used), (iii) the position of the at least one indentation 70 relative to the position of the at least one protrusion 72 at the time the device 10 is assembled and/or at the time the plunger 38 is to be rotated, and (iv) the size of the at least one indentation 70 and the size of the at least one protrusion 72 (e.g., each protrusion and indentation may be 1/36^{th} (i.e., 10 degrees) of the circumference of the inner bore 58 of the cap 24 and the outer circumference of the plunger 38, respectively).

In one exemplary embodiment, the at least one protrusion 72 includes three protrusions substantially equally spaced around the inner bore 58 of the cap (e.g., substantially spaced one hundred twenty degrees apart), and the at least one indentation 70 includes three indentations substantially equally spaced around the outer circumference of the plunger 38 (e.g., substantially spaced one hundred twenty degrees apart). In accordance with this exemplary embodiment, the given number of degrees the plunger 38 is rotated to align the at least one indentation 70 with the at least one protrusion 72 is preferably is about thirty (30) degrees. However, depending on one or more of the factors described above, the given number of degrees may be substantially between five (5) degrees and one hundred twenty (120) degrees.

Referring to Figure 5C, the plunger 38 may then be pushed downwardly into the engaged position so the shaft 42 may pass through the aperture 62 in the cap 24 and pierce the stopper 18. Once the shaft 42 has pierced the stopper 18, the locking mechanism 64 prevents the plunger 38 from being pulled back upwardly to the disengaged position.

The device 10 may include a feedback mechanism that provides a user of the device 10 with feedback regarding operation of the device 10. For example, the feedback may indicate that the shaft 42 has traveled an optimum distance into the stopper 18. Alternatively, or in combination, the feedback may indicate that the plunger 38 has traveled an optimum distance within the cap 24 and that the user should not push the plunger 38 any further. Other examples of the feedback provided by the feedback mechanism are also possible.

The feedback mechanism may be arranged in various configurations. For example, as shown in Figures 2 and 3, the feedback mechanism may comprise (i) a convex bump 55 located on the surface of the second portion 40 of the plunger 38 and extending into the indentation 70, and (ii) a convex bump 57 on the protrusion 72.

The convex bump 55 may be molded as part of the second portion 40. The convex bump 55 may extend 0.2 mm to 1.0 mm (preferably 0.3 mm to 0.6 mm) away from the second portion 40. A widest portion of the convex bump 55 may have a diameter between 0.2 mm to 1.0 mm (preferably 0.3 mm to 0.6 mm).

The convex bump 57 may be molded as part of the indentation 70. The convex bump 57 may extend 0.2 mm to 1.0 mm (preferably 0.3 mm to 0.6 mm) away from the indentation 70. A widest portion of the convex bump 57 may have a diameter between 0.2 mm to 1.0 mm (preferably 0.3 mm to 0.6 mm).

In operation, and by way of example, as the plunger 38 travels in a direction towards the stopper 18, the feedback mechanism provides tactile feedback as the convex bump 55 on the indentation 70 travels past the convex bump 57 on the protrusion 72. The tactile feedback may be felt by the user. Moreover, as the convex bump 55 on the indentation 70 travels past the convex bump 57 on the protrusion 72, a sound may be made such that the feedback mechanism also provides audible feedback.

With reference to Figure 7, the device 10 may include a top 80 which is positioned over the second end 28 of the cap 24 and the plunger 38. The top 80 protects the plunger 38. The top 80 may further include a textured surface 82 for facilitating gripping and removal of the top 80 by a user. Additionally, a tamper-proof mechanism 84 may be located on the device 10 to indicate to a user whether the device 10 has been used. The tamper-proof mechanism 84 may comprise any type of indicator, such as a seal, a holographic label, or a tab, for example.

In operation, the device 10 is in the disengaged position, that is, when the protrusions 72 of the cap 24 and the indentations 70 of the plunger 38 are not aligned, and the shaft 42 of the plunger 38 is not piercing the stopper 18, as shown in Figure 8A. Once the top 80 is removed from the device 10 by a user, the tamper-proof mechanism 84 will be broken. A user may then attach a second receptacle 90, such as a syringe, to the receptacle 12. To activate the device 10, a user may then rotate the plunger 38 about the given number of degrees (e.g., 30 degrees) so the indentations 70 on the plunger 38 align with the protrusions 72 on the cap 24. The plunger 38 may then be pushed in a downward direction toward the stopper 18 into the engaged position, as shown in Figure 8B. The shaft 42 of the plunger 38 may pierce the stopper 18, allowing access to the opening 16 of the receptacle 12. The contents of the second receptacle 90 may then be introduced into the receptacle 12 to mix with the component. The mixed contents may then be pulled back into the second receptacle 90. A needle (not shown) may then be secured to the second receptacle 90, and the complete and active drug may be administered to a patient.

Referring to Figure 9, a second embodiment 110 of the reconstitution device of the present invention is shown. The device 110 may include a receptacle 112 for storing a first component of a pharmaceutical preparation (not shown), such as its active ingredient, for example. The receptacle 112 may include an opening 116 surrounded or partially surrounded by a neck 114. The neck 114 may also include a lip 115. The opening 116 in the neck 114 allows for a second component, such as a liquid (not shown), to be introduced into the receptacle 112 and mix with the first component. A stopper 118 may be positioned in the opening 116 of the neck 114 to block access to the receptacle 112. The stopper 118 may be made of a relatively non-rigid material, such as elastomer. The stopper 118 may include a top portion 120 located against the lip 115, and a bottom portion 122 located within the opening 116 of the neck 114. The top portion 120 may be capable of being perforated, to allow access to the receptacle 112.

The device 110 may further include a cap 124 secured to the neck 114 of the receptacle 112. The cap 124 may have a first end 126, which is secured to the neck 114 of the receptacle 112, and a second end 128 located opposite the first end 126. The first end 126 of the cap 124 may surround at least a portion of the stopper 118. The first end 126 of the cap 124 may include a protrusion 130 for securing the first end 126 to the lip 115 of the receptacle 112.

The first end 126 of the cap 124 may further be secured to the receptacle 112 by a crimp ring 132, which may be positioned in an indentation 125 in the first end 126 of the cap 124. The crimp ring 132 may extend completely or around only a portion of the circumference of the first end 126 of the cap 124. The crimp ring 132 may be made of metal or a polymer with low creep sensitivity. The crimp ring 132 may further include an upper surface 134 for attaching to a top 180, which will be described below.

The device 110 may include a plunger 138 located at the second end 128 of the cap 124. The plunger 138 may include a shaft 142 extending in a direction towards the stopper 118. The shaft 142 may include a pointed end 144 for piercing the top portion 120 of the stopper 118, thereby allowing the shaft 142 access to the receptacle 112. The shaft 142 may further include a filtering mechanism (not shown) similar in structure and function to the filtering mechanism disclosed above with respect to device 10.

The plunger 138 may also include a male element 148 extending in a direction opposite the shaft 142. The male element 148 may be configured to receive a second receptacle (not shown), such as a syringe, for example. The male element 148 may include an inner bore 150 and an outer surface 151. The outer surface 151 may include a thread 152 for mating with the second receptacle.

The shaft 142 of the plunger 138 may also include first and second longitudinal channels (not shown) which establish communication between the receptacle 112 and the inner bore 150 of the male element 148. The longitudinal channels are similar in structure and function to the longitudinal channels described above with respect to device 10. The plunger 138 may further be configured to slide along the outer circumference of the cap 124, as shown in Figure 9.

The cap 124 may further include an inner bore 158 having an annular plate 160 with an aperture 162. The aperture 162 is coaxially aligned with the shaft 142 of the plunger 138. When the plunger 138 moves downward toward the receptacle 112, the shaft 142 moves downwardly through the aperture 162 to pierce the top portion 120 of the stopper 118.

The device 110 may include a mechanism that prevents upward movement of the plunger 138 after downward movement of the plunger 138 towards the stopper 118 has occurred. As an example, the device 110 may include a locking mechanism such as the locking mechanism 64 described above. The locking mechanism may be used to retain the plunger 138 to the device 110.

As another example, the device 110 may include a ratcheting mechanism that prevents upward movement of the plunger 138 after downward movement of the plunger 138 towards the stopper 118 has occurred. The ratcheting mechanism may be used to retain the plunger to the device 110.

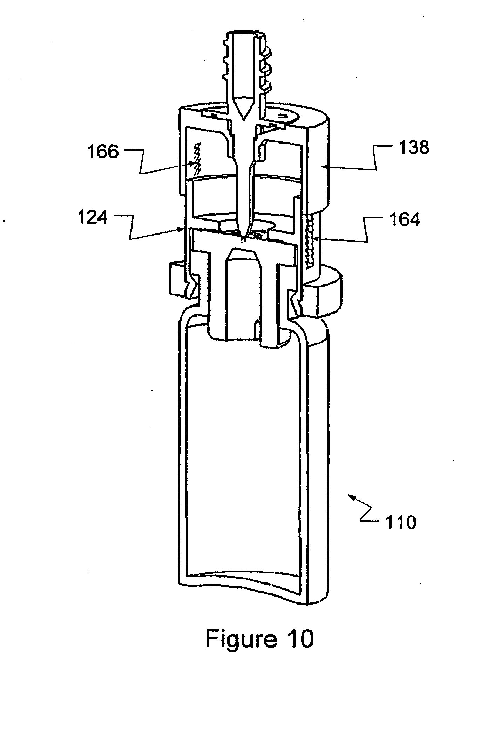

The ratcheting mechanism may include one or more series of ribs and one or more series of teeth. Each of the one or more series of ribs corresponds to one of the series of teeth. Figure 10 shows the device 110 including (i) a series of ribs 164 located on an outer surface of the cap 124, and (ii) a series of teeth 166 located on an inner surface of the plunger 138. The series of ribs 164 corresponds to another set of teeth (not shown) located on the inner surface of the plunger 138. The series of teeth 166 corresponds to another series of ribs (not shown) located on the outer surface of the cap 124.

In this way, as the plunger 138 is moved in a downward direction towards the stopper 118, one or more teeth of a series of teeth (not shown) travel over the series of ribs 164, whereas if upward movement of the plunger 138 is attempted, one or more teeth of the series of teeth (not shown) encounter the series of ribs 164 so as to prevent the one or more teeth as well as the plunger 138 from moving upwards. Similarly, as the plunger 138 is moved in a downward direction towards the stopper 118, one or more teeth of a series of teeth 166 travel over a series of ribs (not shown), whereas if upward movement of the plunger 138 is attempted, one or more teeth of the series of teeth 166 encounter the series of ribs (not shown) so as to prevent the series of teeth 166 as well as the plunger 138 from moving upwards.

The cap 124 and plunger 138 of the device 110 may further include an actuating mechanism similar in structure and function to the actuating mechanism described above with respect to the device 10.

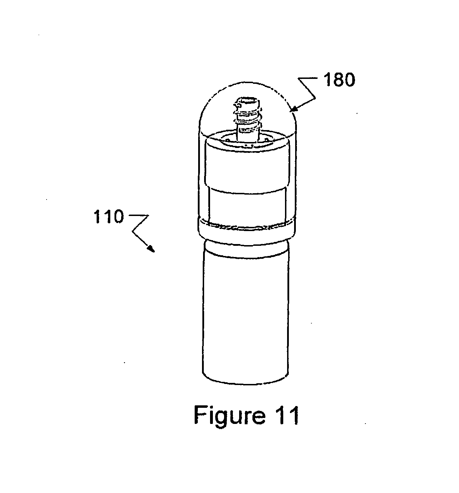

The device 110 may include a top 180, as shown in Figure 11, which fits over the second end 128 of the cap 124 and the plunger 138, and attaches to the crimp ring 132. The top 180 protects the device 110.

Moreover, the top 180 and the crimp ring 132 may be formed as a single piece (i.e., a top and crimp ring combination (not shown)). To accommodate the top and crimp ring combination, the cap 124 may have a first end and a second end, and the first end may be larger in diameter than the second end (similar to the first end 26 of the cap 24 and the second end 28 of the cap 24 shown in Figure 1). In this way, the top and crimp ring combination may slide over the plunger 138 so as to allow the cap 124 to be secured to the receptacle 112. To use the device 110 with the top and crimp ring combination, the top may be broken off (e.g., by twisting the top) and the crimp ring continues to secure the cap 124 to the receptacle 112.

Additionally, a tamper-proof mechanism (not shown) may be located on the device 110 to indicate to a user whether the device has been used. The tamper-proof mechanism may comprise any type of indicator, such as a seal, a holographic label, or a tab, for example.

In operation, the device 110 is in a disengaged position, that is, the shaft 142 of the plunger 138 is not piercing the stopper. Once the top 180 is removed from the device 110, the tamper-proof mechanism will be broken. A user may then attach a second receptacle, such as a syringe, to the receptacle 112. The plunger 138 may then be pushed in a downward direction toward the stopper 118 into an engaged position. The shaft 142 of the plunger 138 may pierce the stopper 118, allowing access to the opening 116 of the receptacle 112. The contents of the second receptacle may then be introduced into the receptacle 112 to mix with the component. The mixed contents may then be pulled back into the second receptacle. A needle (not shown) may then be secured to the second receptacle, and the complete and active drug may be administered to a patient.

While certain features and embodiments of the present invention have been described in detail herein, it is to be understood that the invention encompasses all modifications and enhancements within the scope and spirit of the following claims.

## Claims

1. A reconstitution device (10) comprising;
a receptacle (12);
a cap (24) including a first end (26), a second end (28), and an inner bore (58) having a central aperture (62), the first end (26) being secured to the receptacle (12);
a stopper (18) located between the receptacle (12) and the cap (24), the stopper (18) including a portion capable of being perforated;
a plunger (38) secured to the second end (28) of the cap (24);
a locking mechanism (64) located in the cap (24); and
an actuating mechanism,
**characterized in that**
the actuating mechanism comprises at least one protrusion (72) on the cap (24) and at least one indentation (70) on the plunger (38),
wherein the plunger (38) is rotatable for aligning the at least one protrusion (72) with the at least one indentation (70), and
wherein the plunger (38) is movable in a downward direction when the at least one protrusion (72) is aligned with the at least one indentation (70).

2. The device (10) of claim 1 further comprising a feedback mechanism for indicating to a user when the plunger (38) has pierced the stopper (18).

3. The device (10) of claim 2, wherein the feedback mechanism comprises a convex bump (57) on each of the at least one protrusion (72) on the cap (24), and a convex bump (55) on each of the at least one indentation (70) on the plunger (38).

4. The device (10) of one of the claims 1 to 3, further comprising a feedback mechanism for indicating to a user when the plunger (38) has rotated such that the protrusion (72) aligns with the indentation (70).

5. The device (10) of claim 4, wherein the feedback mechanism provides tactile feedback and audible feedback.

6. The device (10) of claim 2, wherein the plunger (38) further comprises a shaft (42) for perforating the stopper (18).

7. The device (10) of one of the claims 1 to 6, wherein the locking mechanism (64) comprises a thin material including a tongue (66) capable of flexing in a vertical direction.

8. The device (10) of claim 7, wherein the tongue (66) of the locking mechanism (64) further comprises an aperture (68) that is coaxially aligned with the shaft (42) of the plunger (38).

9. The device (10) of one of the claims 1 to 8, wherein the receptacle (12) includes an opening (16) surrounded by a neck (14) for attaching to the first end (26) of the cap (24), the stopper (18) being located in the opening of the neck (14).

10. The device (110) of claim 9, wherein the first end (126) of the cap (124) is secured to the neck (114) of the receptacle (112) by a crimp ring (132).

11. The device (10) of claim 9, wherein the first end (26) of the cap (24) is secured to the receptacle (12) by a C-clip (34).

12. The device (10) of one of the claims 1 to 11, wherein the plunger (38) further includes a male element (48) for receiving a second receptacle.

13. The device (10) of claim 12, wherein the male element (48) includes an inner bore (50) and an outer surface (51) including a thread.

14. The device of claim 12, wherein the second receptacle is a syringe.

15. The device (10) of claims 13, wherein the shaft (42) of the plunger (38) includes an in-line filtering mechanism and a channel (54) for establishing communication between the inside of the receptacle (12) and the inner bore (50) of the male element (48).

16. The device (10) of one of the claims 1 to 15, further comprising a tamper-proof mechanism.

17. The device (10) of one of the claims 1 to 16, further comprising a top (80) surrounding the plunger (38) and the cap (24).

18. The device (10) of claim 1, wherein the plunger (38) is adapted to slide along the inner bore (58) of the cap (24).

19. The device (110) of one of the claims 1 to 18, wherein the plunger (138) is adapted to slide along an outer surface of the cap (124).

20. The device (10) of one of the claims 1 to 19, wherein the plunger (38) has a top surface (41) including direction markers (74) and alignment markers (76).

21. The device (10) of one of the claims 1 to 20, wherein the top surface (41) comprises a dome shape.

22. The device (10) of one of the claims 1 to 21, wherein the top surface (41) is angled.

## Patentansprüche

1. Rekonstitutionsvorrichtung (10) mit einem Behältnis (12),
einer Kappe (24) mit einem ersten Ende (26), einem zweiten Ende (28) und einer inneren Bohrung (58) mit einer mittleren Öffnung (62), wobei das erste Ende (26) am Behältnis (12) befestigt ist,
einem Stopfen (18), der zwischen dem Behältnis (12) und der Kappe (24) angeordnet ist und einen Abschnitt aufweist, der perforiert werden kann, einem Stößel (38), der am zweiten Ende (28) der Kappe (24) befestigt ist,
einem Verriegelungsmechanismus (64), der in der Kappe (24) angeordnet ist, und
einem Betätigungsmechanismus,
**dadurch gekennzeichnet, dass**
der Betätigungsmechanismus mindestens einen Vorsprung (72) an der Kappe (24) und mindestens eine Vertiefung (70) am Stößel (38) umfasst,
wobei der Stößel (38) gedreht werden kann, um den mindestens einen Vorsprung (72) auf die mindestens eine Vertiefung (70) auszurichten, und
wobei der Stößel (38) in eine nach unten gehende Richtung bewegt werden kann, wenn der mindestens eine Vorsprung (72) auf die mindestens eine Vertiefung (70) ausgerichtet ist.

2. Vorrichtung (10) nach Anspruch 1, ferner mit einem Rückkopplungsmechanismus, um einem Benutzer gegenüber anzugeben, wann der Stößel (38) den Stopfen (18) durchstochen hat.

3. Vorrichtung (10) nach Anspruch 2, wobei der Rückkopplungsmechanismus einen konvexen Buckel (57) an jedem des mindestens einen Vorsprungs (72) an der Kappe (24) und einen konvexen Buckel (55) an jeder der mindestens einen Vertiefung (70) am Stößel (38) umfasst.

4. Vorrichtung (10) nach einem der Ansprüche 1 bis 3, ferner mit einem Rückkopplungsmechanismus, um einem Benutzer gegenüber anzugeben, wann der Stößel (38) sich so gedreht hat, dass der Vorsprung (72) auf die Vertiefung (70) ausgerichtet ist.

5. Vorrichtung (10) nach Anspruch 4, wobei der Rückkopplungsmechanismus haptische Rückkopplung und hörbare Rückkopplung bereitstellt.

6. Vorrichtung (10) nach Anspruch 2, wobei der Stößel (38) ferner einen Schaft (42) zum Perforieren des Stopfens (18) umfasst.

7. Vorrichtung (10) nach einem der Ansprüche 1 bis 6, wobei der Verriegelungsmechanismus (64) ein dünnes Material einschließlich einer Zunge (66) umfasst, das sich in einer vertikalen Richtung biegen kann.

8. Vorrichtung (10) nach Anspruch 7, wobei die Zunge (66) des Verriegelungsmechanismus (64) ferner eine Öffnung (68) umfasst, die koaxial auf den Schaft (42) des Stößels (38) ausgerichtet ist.

9. Vorrichtung (10) nach einem der Ansprüche 1 bis 8, wobei das Behältnis (12) eine Öffnung (16) aufweist, die zum Anbringen am ersten Ende (26) der Kappe (24) von einer Verengung (14) umgeben ist, wobei der Stopfen (18) in der Öffnung der Verengung (14) angeordnet ist.

10. Vorrichtung (110) nach Anspruch 9, wobei das erste Ende (126) der Kappe (124) über einen Crimp-Ring (132) an der Verengung (114) des Behältnisses (112) befestigt ist.

11. Vorrichtung (10) nach Anspruch 9, wobei das erste Ende (26) der Kappe (24) über einen C-Clip (34) an dem Behältnis (12) befestigt ist.

12. Vorrichtung (10) nach einem der Ansprüche 1 bis 11, wobei der Stößel (38) ferner ein Steckelement (48) zur Aufnahme eines zweiten Behältnisses aufweist.

13. Vorrichtung (10) nach Anspruch 12, wobei das Steckelement (48) eine innere Bohrung (50) und eine Außenfläche (51) mit einem Gewinde aufweist.

14. Vorrichtung nach Anspruch 12, wobei das zweite Behältnis eine Spritze ist.

15. Vorrichtung (10) nach Anspruch 13, wobei der Schaft (42) des Stößels (38) einen In-Line-Filtermechanismus und einen Kanal (54) zur Herstellung einer Verbindung zwischen dem Inneren des Behältnisses (12) und der inneren Bohrung (50) des Steckelements (48) aufweist.

16. Vorrichtung (10) nach einem der Ansprüche 1 bis 15, ferner mit einem Originalitätssicherungsmechanismus.

17. Vorrichtung (10) nach einem der Ansprüche 1 bis 16, ferner mit einem Oberteil (80), das den Stößel (38) und die Kappe (24) umgibt.

18. Vorrichtung (10) nach Anspruch 1, wobei der Stößel (38) geeignet ist, die innere Bohrung (58) der Kappe (24) entlang zu gleiten.

19. Vorrichtung (110) nach einem der Ansprüche 1 bis 18, wobei der Stößel (138) geeignet ist, an einer Außenfläche der Kappe (124) entlang zu gleiten.

20. Vorrichtung (10) nach einem der Ansprüche 1 bis 19, wobei der Stößel (38) eine obere Fläche (41) mit Richtungsmarkierungen (74) und Ausrichtungsmarkierungen (76) hat.

21. Vorrichtung (10) nach einem der Ansprüche 1 bis 20, wobei die obere Fläche (41) eine Kuppelform umfasst.

22. Vorrichtung (10) nach einem der Ansprüche 1 bis 21, wobei die obere Fläche (41) abgewinkelt ist.

## Revendications

1. Dispositif de reconstitution (10) comprenant :
un récipient (12) ;
un capuchon (24) comprenant une première extrémité (26), une seconde extrémité (28) et un alésage intérieur (58) comportant une ouverture centrale (62), la première extrémité (26) étant solidement fixée au récipient (12) ;
un bouchon (18) situé entre le récipient (12) et le capuchon (24), le bouchon (18) comprenant une partie pouvant être perforée ;
un piston (38) solidement fixé à la seconde extrémité (28) du capuchon (24) ;
un mécanisme de verrouillage (64) situé dans le capuchon (24) ; et
un mécanisme d'actionnement,
**caractérisé en ce que** le mécanisme d'actionnement comprend au moins une protubérance (72) sur le capuchon (24) et une échancrure (70) sur le piston (38),
dans lequel le piston (38) est rotatif afin d'aligner ladite protubérance (72) avec ladite échancrure (70), et
dans lequel le piston (38) est mobile dans le sens vers le bas quand ladite protubérance (72) est alignée avec ladite échancrure (70).

2. Dispositif (10) selon la revendication 1, comprenant un mécanisme de remontée de l'information pour indiquer à un utilisateur quand le piston (38) a percé le bouchon (18).

3. Dispositif (10) selon la revendication 2, dans lequel le mécanisme de remontée de l'information comprend une bosse convexe (57) sur chacune desdites protubérances (72) sur le capuchon (24) et une bosse convexe (55) sur chacune desdites échancrures (70) sur le piston (38).

4. Dispositif (10) selon l'une quelconque des revendications 1 à 3, comprenant en outre un mécanisme de remontée de l'information pour indiquer à un utilisateur quand le piston (38) a tourné de telle sorte que la protubérance (72) est alignée avec l'échancrure (70).

5. Dispositif (10) selon la revendication 4, dans lequel le mécanisme de remontée de l'information fournit des retours tactiles et des retours audibles.

6. Dispositif (10) selon la revendication 2, dans lequel le piston (38) comprend un axe (42) pour perforer bouchon (18).

7. Dispositif (10) selon l'une quelconque des revendications 1 à 6, dans lequel le mécanisme de verrouillage (64) comprend une matière mince comprenant une languette (66) capable de fléchir dans le sens vertical.

8. Dispositif (10) selon la revendication 7, dans lequel la languette (66) du mécanisme de verrouillage (64) comprend en outre une ouverture (68) qui est en alignement coaxial avec l'axe (42) du piston (38).

9. Dispositif (10) selon l'une quelconque des revendications 1 à 8, dans lequel le récipient (12) comprend une ouverture (16) entourée par un collet (14) pour se fixer à la première extrémité (26) du capuchon (24), le bouchon (18) étant situé dans l'ouverture du collet (14).

10. Dispositif (110) selon la revendication 9, dans lequel la première extrémité (126) du capuchon (124) est solidement fixée au collet (114) du récipient (112) par une bague de sertissage (132).

11. Dispositif (10) selon la revendication 9, dans lequel la première extrémité (26) du capuchon (24) est solidement fixée au récipient (12) par un circlip (34).

12. Dispositif (10) selon l'une quelconque des revendications 1 à 11, dans lequel le piston (38) comprend en outre un élément mâle (48) pour recevoir un second récipient.

13. Dispositif (10) selon la revendication 12, dans lequel l'élément mâle (48) comprend un alésage intérieur (50) et une surface extérieure (51) comprenant un filetage.

14. Dispositif selon la revendication 12, dans lequel le second récipient est une seringue.

15. Dispositif (10) selon la revendication 13, dans lequel l'axe (42) du piston (38) comprend un mécanisme de filtration en ligne et un canal (54) pour établir une communication entre l'intérieur du récipient (12) et l'alésage intérieur (50) de l'élément mâle (48).

16. Dispositif (10) selon l'une quelconque des revendications 1 à 15, comprenant en outre un mécanisme inviolable.

17. Dispositif (10) selon l'une quelconque des revendications 1 à 16, comprenant en outre un couvercle (80) entourant le piston (38) et le capuchon (24).

18. Dispositif (10) selon la revendication 1, dans lequel le piston (38) est apte à coulisser le long de l'alésage intérieur (58) du capuchon (24).

19. Dispositif (110) selon l'une quelconque des revendications 1 à 18, dans lequel le piston (138) est apte à coulisser le long de la surface extérieure du capuchon (124).

20. Dispositif (10) selon l'une quelconque des revendications 1 à 19, dans lequel le piston (38) comporte une surface supérieure (41) comprenant des repères de sens (74) et des repères d'alignement (76).

21. Dispositif (10) selon l'une quelconque des revendications 1 à 20, dans lequel la surface supérieure (41) est en forme de dôme.

22. Dispositif (10) selon l'une quelconque des revendications 1 à 21, dans lequel la surface supérieure (41) est inclinée.
